# FASCICULE DE BREVET EUROPEEN

(11) **EP 0 583 347 B1**
(45) Date de publication et mention de la délivrance du brevet: **19.07.1995**
(21) Numéro de dépôt: 92910782.9
(22) Date de dépôt: 30.04.1992
(51) Int. Cl.: A61K 7/043

(54) **VERNIS A ONGLES INCOLORE OU COLORE CONTENANT DES FIBRES D'ARAMIDE**
FARBIGE ODER FARBLOSE ARAMIDFASERN ENTHALTENDE NAGELLACKE
COLOURED OR COLOURLESS NAIL VARNISH CONTAINING ARAMIDE FIBRES

(30) Priorité: 02.05.1991 FR 9105395
(43) Date de publication de la demande: 23.02.1994
(73) Titulaire: L'OREAL, 75008 Paris (FR)
(72) Inventeur: FRANKFURT, Christopher, C., Old Bridge, NJ 08867 (US); FARER, Alan, M., Morganville, NJ 07751 (US); PENICNAK, A., John, Mountain Lakes, NJ 07040 (US)
(74) Mandataire: Stalla-Bourdillon, Bernard
(86) Numéro de dépôt international: FR9200401
(87) Numéro de publication internationale: WO9219282

(56) Documents cités:
- FR-A- 1 529 329
- FR-A- 2 578 741
- GB-A- 1 177 420
- US-A- 4 873 077

## Description

La présente invention a pour objet un vernis à ongles incolore ou coloré, contenant, en plus des ingrédients usuels, des fibres d'aramide.

Parmi les principales caractéristiques que doivent posséder les vernis à ongles, on doit tout particulièrement mentionner une bonne adhérence sur la surface de l'ongle ainsi qu'une certaine flexibilité et résistance du film, ceci en vue d'éviter sa fragilité pouvant conduire à une craquelure du vernis.

De façon générale, on utilise à l'heure actuelle, pour cdaférer une bonne adhérence, des résines modifiées et des plastifiants ce qui conduit à une bonne flexibilité du vernis.

Après d'importantes recherches, on vient maintenant de constater qu'une bonne adhérence d'un vernis incolore ou coloré sur la surface de l'ongle ainsi qu'une bonne résistance pouvaient être obtenues en incorporant certaines fibres.

Il a déjà été proposé dans le brevet britannique 1,177,420 des vernis pour le renforcement et la réparation des ongles cassés contenant de 0,5 à 12 % de certaines fibres telles que par exemple des fibres de soie, de coton, de nylon, de polyacrylonitrile, de polyéthylènethéréphtalate et d'autres polymères ou copolymères.

Selon ce brevet, les fibres présentes dans le vernis agissent en tant qu'agent de renforcement de l'ongle mais ne favorisent en aucune manière l'adhérence et la résistance du vernis sur l'ongle.

La présente invention a donc pour objet, à titre de produit industriel nouveau, un vernis à ongles incolore ou coloré contenant dans un système solvant pour vernis, une substance filmogène, une résine et un agent plastifiant, ledit vernis contenant en outre de 0,01 à 0,5 % en poids de fibres d'aramide.

Par l'expression "fibres d'aramide", on doit entendre des fibres de poly(paraphénylène-téréphtalamide) obtenues par polycondensation de la paraphénylènediamine et du chlorure de téréphtalyle.

Parmi les fibres d'aramide qui peuvent être utilisées dans les vernis selon l'invention, on peut notamment citer les fibres connues sous les dénominations commerciales de "KEVLAR"® en particulier de "KEVLAR DRY PULP"® vendues par la Société DUPONT DE NEMOURS, et "ARENKA"® vendues par la Société ENKA GLANZSTOFF.

La taille des fibres est généralement comprise entre environ 2 et 4 mm.

Selon une forme de réalisation particulière de l'invention, le vernis contient de préférence de 0,05 à 0,2 % en poids de fibres d'aramide.

La concentration en fibres d'aramide est généralement plus élevée dans un vernis incolore que dans un vernis coloré.

Dans un vernis incolore ou vernis de base, les fibres d'aramide confèrent un aspect légèrement rugueux au film déposé permettant une meilleure adhérence du vernis à ongles coloré appliqué ultérieurement, sans que la brillance du film coloré ne soit modifiée.

Par ailleurs, l'application du vernis coloré se révèle beaucoup plus aisée, celle-ci étant homogène sur l'ensemble de la surface de l'ongle.

Le système solvant du vernis selon l'invention est généralement présent en une proportion comprise entre 55 et 90 % en poids par rapport au poids total du vernis.

Ce système solvant est essentiellement constitué par un mélange de divers solvants organiques volatils, ceci en vue d'obtenir des temps de séchage relativement courts.

Parmi ces solvants, on peut citer l'acétone, l'acétate d'éthyle, l'acétate de butyle, l'acétate de méthoxy-2 éthyle, la méthyléthylcétone, la méthylisobutylcétone, l'acétate de méthyle, l'acétate d'amyle et l'acétate d'isopropyle.

Le système solvant peut comprendre également un diluant qui est de préférence un hydrocarbure linéaire ou ramifié saturé, tel que l'hexane ou l'octane ou encore un hydrocarbure aromatique tel que le toluène ou le xylène dans une proportion généralement comprise entre 10 et 30 % par rapport au poids total du vernis.

Le système solvant peut également contenir d'autres solvants volatils tels que de l'éthanol, du n-butanol, du n-propanol, de l'isopropanol ou leurs mélanges.

L'agent filmogène est généralement présent dans le vernis selon l'invention à une concentration comprise entre 5 et 20 % et de préférence entre 10 et 20 % en poids par rapport au poids total du vernis.

Parmi les agents filmogènes particulièrement préférés on doit citer les nitrocelluloses du type "RS" ou "SS" et en particulier la nitrocellulose type 1/4 seconde RS, la nitrocellulose type 1/2 seconde RS, la nitrocellulose type 1/2 seconde SS et la nitrocellulose type 3/4 seconde RS.

On utilise de préférence selon l'invention la nitrocellulose 1/2 seconde SS.

Comme agent filmogène, on peut également utiliser selon l'invention les dérivés polyvinyliques tels que le butyral de polyvinyle ainsi que les copolymères décrits dans les brevets français n° 2.478.998 2.499.851 et 2.617.043.

Dans le vernis selon l'invention, la résine est généralement présente en une proportion comprise entre 0,5 et 10 % en poids par rapport au poids total du vernis.

Parmi les nombreuses résines utilisables, on préfère employer selon l'invention les résines du type arylsulfonamide formaldéhyde ou arylsulfonamide epoxy notamment la résine toluène sulfonamide formaldéhyde plus connue sous les dénominations commerciales de "SANTOLITE MHP"® et "SANTOLITE MS 80%",® la première étant la plus dure alors que la seconde conduit à la formation de film de plus grande flexibilité.

Ces résines permettent d'augmenter le pouvoir filmogène et améliorent le brillant ainsi que l'adhérence.

Selon l'invention, l'agent plastifiant est généralement présent dans le vernis à une concentration comprise entre 2 et 10 % en poids par rapport au poids total du vernis.

Les agents plastifiants permettent de régler la flexibilité du film sans affaiblir sa résistance aux forces physiques. Parmi les agents plastifiants susceptibles d'être utilisés dans les vernis selon l'invention, on peut notamment citer : le phosphate de tricrésyle, le benzoate de benzyle, le phosphate de tributyle, l'acétyl ricinoléate de butyle, l'acétyl ricinoléate de glycéryle, le phtalate de dibutyle, le glycolate de butyle, le phtalate de dioctyle, le stéarate de butyle, le phosphate de tributoxyéthyle, le phosphate de triphényle, le citrate de triéthyle, le citrate de tributyle, l'acétyl citrate de tributyle, l'acétyl citrate de triéthyl-2 hexyle, le tartrate de dibutyle, le phtalate de diméthoxyéthyle, le phtalate de di-isobutyle, le phtalate de diamyle, le camphre, le triacétate de glycérol et leurs mélanges.

Si une des formes de réalisation préférée de l'invention est un vernis incolore, celui-ci peut néanmoins être coloré à l'aide d'au moins un agent colorant de nature organique ou inorganique.

Parmi les agents colorants organiques, on peut citer les D et C RED n° 10, 11, 12 et 13, le D et C RED n° 7, les D et C RED n° 5 et 6, le D et C RED n° 34, des laques telles que la laque D et C YELLOW n° 5 et la laque D et C RED n° 2.

Parmi les agents colorants inorganiques, on peut citer le bioxyde de titane, l'oxychlorure de bismuth, l'oxyde de fer brun et les oxydes de fer rouge. On peut également citer comme agent colorant la guanine.

Selon cette forme de réalisation, les agents colorants sont généralement présents en une proportion comprise entre 0,01 et 2 % en poids par rapport au poids total du vernis.

L'emploi de fibres d'aramide dans les vernis selon l'invention présente par ailleurs l'avantage de pouvoir se passer partiellement ou totalement de produits permettant d'éviter la sédimentation des agents colorants tels que par exemple la "BENTONE 27"® ou la "BENTONE 38"®, les fibres d'aramide agissant en tant qu'agents thixotropes.

Les vernis à ongles selon l'invention peuvent en outre contenir des adjuvants couramment utilisés dans les vernis à ongles. Parmi ces adjuvants, on peut citer les filtres UV tels que la benzophénone et le 2-cyano-3,3-diphénylacrylate d'éthyle.

L'obtention des vernis incolores ou colorés selon l'invention se fait selon les méthodes classiques, toutefois, il convient d'ajouter les fibres d'aramide au dernier moment avant l'étape d'agitation finale.

On va maintenant donner à titre d' illustration plusieurs exemples de vernis à ongles selon l'invention.

| EXEMPLE 1 - Vernis à ongles non coloré | |
|---|---|
| | % en poids |
| - Toluène | 21,93 |
| - Acétate de n-butyle | 20,03 |
| - Acétate d'éthyle | 18,56 |
| - Nitrocellulose 1/2 SS | 15,44 |
| - Alcool isopropylique | 8,21 |
| - Acétyl citrate de tributyle | 6,40 |
| - Butyral de polyvinyle | 2,60 |
| - Silice hydratée | 2,35 |
| - Résine toluène sulfonamide formaldéhyde | 1,10 |
| - Bentone 27 ® | 1,00 |
| - 2-cyano-3,3-diphénylacrylate d'éthyle | 1,00 |
| - Camphre | 0,90 |
| - Bioxyde de titane | 0,24 |
| - Fibres d'aramide (KEVLAR® de la Société DUPONT) | 0,15 |
| - Acide citrique | 0,05 |
| - Benzophénone | 0,04 |

| EXEMPLE 2 - Vernis à ongles non coloré | |
|---|---|
| - Toluène | 21,30 |
| - Acétate de n-butyle | 19,40 |
| - Acétate d'éthyle | 18,56 |
| - Nitrocellulose 1/2 SS | 17,02 |
| - Alcool isopropylique | 8,21 |
| - Acétyl citrate de tributyle | 6,40 |
| - Butyral de polyvinyle | 2,60 |
| - Silice hydratée | 2,35 |
| - Résine toluène sulfonamide époxy | 1,10 |
| - Bentone 27 | 1,00 |
| - 2-cyano-3,3 diphénylacrylate d'éthyle | 1,00 |
| - Camphre | 0,90 |
| - Fibres d'aramide (KEVLAR® de la Société DUPONT) | 0,07 |
| - Acide citrique | 0,05 |
| - Benzophénone | 0,04 |

| EXEMPLE 3 - Vernis à ongles coloré | |
|---|---|
| - Toluène | 21,15 |
| - Acétate de n-butyle | 19,26 |
| - Acétate d'éthyle | 18,43 |
| - Nitrocellulose 1/2 SS | 16,90 |
| - Alcool isopropylique | 8,15 |
| - Acétyl citrate de tributyle | 6,36 |
| - Butyral de polyvinyle | 2,58 |
| - Silice hydratée | 2,33 |
| - Bentone 27 ® | 1,06 |
| - Résine toluène sulfonamide époxy | 1,04 |
| - 2-cyano-3,3-diphénylacrylate d'éthyle | 0,99 |
| - Camphre | 0,89 |
| - D & C Red n° 6, barium lake | 0,36 |
| - D & C Red n° 7, calcium lake | 0,21 |
| - Bioxyde de titane | 0,07 |
| - Fibres d'aramide (KEVLAR® de la Société DUPONT) | 0,07 |
| - Guanine | 0,06 |
| - Acide citrique | 0,05 |
| - Benzophénone | 0,04 |

## Revendications

1. Vernis à ongles, incolore ou coloré, contenant dans un système solvant pour vernis, une substance filmogène, une résine et un agent plastifiant caractérisé par le fait que ledit vernis contient de 0,01 à 0,5 % en poids de fibres d'aramide.

2. Vernis à ongles selon la revendication 1, caractérisé par le fait que les fibres d'aramide sont des fibres de poly(paraphénylène-téréphtalamide).

3. Vernis à ongles selon la revendication 1 ou 2, caractérisé par le fait que les fibres d'aramide ont une taille comprise entre 2 et 4 mm.

4. Vernis à ongles selon l'une quelconque des revendications 1 à 3, caractérisé par le fait qu'il contient de 0,05 à 0,2 % en poids de fibres d'aramide.

5. Vernis à ongles selon la revendication 1, caractérisé par le fait que le système solvant est présent en une proportion comprise entre 55 et 90 % en poids par rapport au poids total du vernis.

6. Vernis à ongles selon la revendication 1, caractérisé par le fait que l'agent filmogène est présent à une concentration comprise entre 5 et 20 % en poids par rapport au poids total du vernis.

7. Vernis à ongles selon la revendication 1 ou 6, caractérisé par le fait que l'agent filmogène est de la nitrocellulose 1/2 seconde SS.

8. Vernis à ongles selon la revendication 1, caractérisé par le fait que la résine est présente en une proportion comprise entre 0,5 et 10 % en poids par rapport au poids total du vernis.

9. Vernis à ongles selon la revendication 1 ou 8, caractérisée par le fait que la résine est choisie parmi les résines aryl sulfonamide formaldéhyde et aryl sulfonamide époxy.

10. Vernis à ongles selon la revendication 1, caractérisé par le fait que l'agent plastifiant est présent à une concentration comprise entre 2 et 10 % en poids par rapport au poids total du vernis.

11. Vernis à ongles selon l'une quelconque des revendications précédentes, caractérisé par le fait qu'il contient au moins un agent colorant de nature organique ou inorganique.

12. Vernis à ongles selon la revendication 11, caractérisé par le fait que l'agent colorant est présent à une concentration comprise entre 0,01 et 2 % par rapport au poids total du vernis.

## Claims

1. Coloured or clear nail varnish containing, in a varnish solvent system, a film-forming substance, a resin and a plasticizer, characterized in that the said varnish contains from 0.01 to 0.5% by weight of aramid fibres.

2. Nail varnish according to Claim 1, characterized in that the aramid fibres are poly(para-phenylene terephthalamide) fibres.

3. Nail varnish according to Claim 1 or 2, characterized in that the aramid fibres have a size of between 2 and 4 mm.

4. Nail varnish according to any one of Claims 1 to 3, characterized in that it contains from 0.05 to 0.2% by weight of aramid fibres.

5. Nail varnish according to Claim 1, characterized in that the solvent system is present in a proportion of between 55 and 90% by weight with respect to the total weight of the varnish.

6. Nail varnish according to Claim 1, characterized in that the film-forming agent is present at a concentration of between 5 and 20% by weight with respect to the total weight of the varnish.

7. Nail varnish according to Claim 1 or 6, characterized in that the film-forming agent is SS 1/2 second nitrocellulose.

8. Nail varnish according to Claim 1, characterized in that the resin is present in a proportion of between 0.5 and 10% by weight with respect to the total weight of the varnish.

9. Nail varnish according to Claim 1 or 8, characterized in that the resin is chosen from arylsulphonamide-formaldehyde and arylsulphonamide epoxy resins.

10. Nail varnish according to Claim 1, characterized in that the plasticizer is present at a concentration of between 2 and 10% by weight with respect to the total weight of the varnish.

11. Nail varnish according to any one of the preceding claims, characterized in that it contains at least one colouring agent of organic or inorganic nature.

12. Nail varnish according to Claim 11, characterized in that the colouring agent is present at a concentration of between 0.01 and 2% with respect to the total weight of the varnish.

## Patentansprüche

1. Farbloser oder gefärbter Nagellack mit einem Gehalt an einem Lösungsmittelsystem für Nagellacke, einer filmbildenden Substanz, einem Kunstharz sowie einem Weichmacher, dadurch gekennzeichnet, daß der Lack einen Gehalt von 0,01 Gew.-% bis 0,5 Gew.-% Aramidfasern aufweist.

2. Nagellack gemäß Anspruch 1, dadurch gekennzeichnet, daß die Aramidfasern als Poly-(paraphenylenterephthalamid)-fasern vorliegen.

3. Nagellack gemäß Anspruch 1 oder 2, dadurch gekennzeichnet, daß die Aramidfasern eine Länge von 2 bis 4 mm aufweisen.

4. Nagellack gemäß einem der Ansprüche 1 bis 3, dadurch gekennzeichnet, daß er 0,05 Gew.-% bis 0,2 Gew.-% Aramidfasern enthält.

5. Nagellack gemäß Anspruch 1, dadurch gekennzeichnet, daß das Lösungsmittelsystem in einem Mengenverhältnis von 55 Gew.-% bis 90 Gew.-% in bezug auf das Gesamtgewicht des Lacks vorliegt.

6. Nagellack gemäß Anspruch 1, dadurch gekennzeichnet, daß die filmbildende Substanz in einer Konzentration zwischen 5 Gew.-% und 20 Gew.-% in bezug auf das Gesamtgewicht des Lacks vorliegt.

7. Nagellack gemäß Anspruch 1 oder 6, dadurch gekennzeichnet, daß die filmbildende Substanz ein Cellulosenitrat vom Typ 1/2 Sekunde SS darstellt.

8. Nagellack gemäß Anspruch 1, dadurch gekennzeichnet, daß das Kunstharz in einem Mengenverhältnis zwischen 0,5 Gew.-% und 10 Gew.-% in bezug auf das Gesamtgewicht des Lacks vorhanden ist.

9. Nagellack gemäß Anspruch 1 oder 8, dadurch gekennzeichnet, daß das Kunstharz unter Arylsulfonamidformaldehyd- oder Arylsulfonamidepoxyharz ausgewählt ist.

10. Nagellack gemäß Anspruch 1, dadurch gekennzeichnet, daß der Weichmacher in einer Konzentration zwischen 2 Gew.-% und 20 Gew.-% in bezug auf das Gesamtgewicht des Lacks vorhanden ist.

11. Nagellack gemäß einem der vorstehenden Ansprüche, dadurch gekennzeichnet, daß er mindestens einen organischen oder anorganischen Farbstoff enthält.

12. Nagellack gemäß Anspruch 11, dadurch gekennzeichnet, daß der Farbstoff in einer Konzentration zwischen 0,01 Gew.-% und 2 Gew.-% in bezug auf das Gesamtgewicht des Lacks vorhanden ist.
